# FASCICULE DE BREVET EUROPEEN

(11) **EP 0 986 411 B1**
(45) Date de publication et mention de la délivrance du brevet: **03.08.2005**
(21) Numéro de dépôt: 98929520.9
(22) Date de dépôt: 09.06.1998
(51) Int. Cl.: A61M 5/24, A61M 5/32

(54) **SERINGUE D'INJECTION PERFECTIONNEE, COMPORTANT DES MOYENS D'ASPIRATION**
PERFEKTIONIERTE INJEKTIONSSPRITZE MIT ANSAUGVORRICHTUNG
IMPROVED INJECTION SYRINGE, COMPRISING SUCKING MEANS

(30) Priorité: 09.06.1997 FR 9707097
(43) Date de publication de la demande: 22.03.2000
(73) Titulaire: SOFIC, Aussillon, 81200 Mazamet (FR)
(72) Inventeur: GRANIER, Patrick, F-81200 Mazamet (FR)
(74) Mandataire: Morelle, Guy Georges Alain
(86) Numéro de dépôt international: PCT/FR1998/001171
(87) Numéro de publication internationale: WO 1998/056437

(56) Documents cités:
- WO-A-94/16752
- WO-A-97/40875
- US-A- 3 931 815
- US-A- 5 207 646

## Description

La présente invention se rapporte aux seringues, dispositifs, et modules d'injection de type à usage unique, notamment mais non exclusivement pour le domaine dentaire.

De telles seringues sont connues des fabricants de ces matériels, des praticiens et des patients et comportent généralement un corps muni d'une aiguille à une première extrémité de celui-ci, l'aiguille comportant une partie extérieure s'étendant à l'extérieur du corps et une partie intérieure s'étendant à l'intérieur du corps, l'aiguille formant ainsi une extrémité extérieure au corps et une extrémité intérieure au corps, des moyens de préhension du corps de seringue, à une deuxième extrémité de celui-ci opposée à la première extrémité, une tige d'injection mobile, une cartouche amovible contenant un produit à injecter, disposée à l'intérieur du corps entre l'extrémité intérieure de l'aiguille et la deuxième extrémité du corps dans une position de repos, cette cartouche étant apte à se déplacer de la position de repos vers la première extrémité du corps sous une pression de la tige d'injection, lors d'une injection.

Les dispositifs évoqués plus haut sont constitués des seringues décrites ci-dessus, donc destinés à fonctionner de manière conventionnelle en coopération avec une cartouche préremplie de produit à injecter, mais sont commercialisés sans cette cartouche, afin de permettre au praticien d'y introduire celle de son choix, en fonction de ses besoins.

Les modules également évoqués plus haut, sont constitués par une partie des dispositifs décrits ci-dessus, qui peuvent être commercialisés sans les moyens de préhension et sans la tige d'injection, ces derniers étant alors constitués par des moyens de préhension et une tige amovibles et réutilisables dont peut disposer l'utilisateur, seul le module (et la cartouche) étant dans ce cas à usage unique et jetés après utilisation.

De tels seringues, dispositifs et modules fonctionnent parfaitement. Toutefois, comme il est connu dans le domaine dentaire notamment, le praticien qui doit réaliser une injection de produits anesthésiques par exemple, doit s'assurer que l'extrémité extérieure de la seringue ne débouche pas dans un vaisseau sanguin de la cavité buccale. Pour cela, la pratique consiste à opérer une légère aspiration au moyen de la seringue d'injection afin de vérifier que du sang ne vienne pas colorer le produit à injecter, le cas échéant.

Cette opération réalisée lorsque l'aiguille a pénétré dans la chair nécessite une manoeuvre délicate et peu pratique avec les dispositifs de l'art antérieur, par une traction précisément dosée sur la tige d'injection tout en maintenant le corps immobile, cette traction ne devant pas être trop forte pour éviter l'introduction éventuelle d'une quantité de sang trop élevée le cas échéant, mais suffisante pour permettre d'assurer la vérification de manière certaine.

On connait notamment avec la demande française n° 2 696 096 une seringue permettant d'améliorer l'opération d'aspiration par l'introduction d'un "amortisseur" de déplacement de la tige, en quelque sorte. Cette demande enseigne une seringue comportant une tige munie à une extrémité d'une tête de section complémentaire de la section intérieure de la cartouche, ladite tête étant destinée à appuyer sur le bouchon mobile de la cartouche pour permettre l'expulsion du produit hors de celle-ci par l'aiguille d'injection, cette tête étant apte à coulisser sur les parois internes de la cartouche et présentant un conduit de fuite reliant le volume délimité entre la tête et le bouchon à l'extérieur de la cartouche, ce conduit de fuite ayant une perte de charge importante.

Ainsi, l'air emprisonné dans un premier temps dans le volume ci-dessus est expulsé par le conduit de fuite, de manière à permettre à la tête d'entrer en contact avec le bouchon. Dans un deuxième temps, l'utilisateur par une faible traction exercée sur la tige va entrainer sur une courte distance la tête en direction de la position avant utilisation ; le bouchon adhérent encore momentanément à la paroi interne de la cartouche, il va alors être crée un vide partiel entre la tête et le bouchon de manière à contraindre celui-ci à quitter sa position et à venir à nouveau en contact avec la tête. Le déplacement du bouchon en direction de sa position avant utilisation va générer ainsi un léger phénomène d'aspiration, suffisant toutefois pour autoriser l'introduction éventuelle de quelques gouttes de sang dans la cartouche le cas échéant.

Ce dispositif qui fonctionne parfaitement et améliore de beaucoup les dispositifs antérieurs nécessitant une manoeuvre complexe de la tige par rotation et traction combinées, demande toutefois au praticien d'exercer une traction pour créer un phénomène d'aspiration.

On connaît le document WO 94/16752 qui se rapporte à un dispositif d'auto-injection possédant un manchon tubulaire dans lequel est disposée de manière axiale une cartouche fermée à son extrémité avant par une membrane et à son extrémité arrière par un piston. Une aiguille d'injection est montée coulissante de manière axiale dans la cartouche. Un capuchon d'actionnement tubulaire, monté prisonnier, est guidé de manière coulissante sur l'extrémité arrière du manchon et permet d'actionner le dispositif via une tige qui agit sur le piston et s'étend à partir du fond du capuchon jusque dans le manchon. Ce dispositif comporte entre la membrane de la cartouche et un obturateur placé dans le manchon et portant l'aiguille, un capuchon protecteur destiné à maintenir une partie de l'aiguille dans des conditions de stérilité, et un ressort d'écartement qui a pour fonction de maintenir la cartouche écartée de l'extrémité intérieure de l'aiguille afin d'éviter un perçage accidentel de la membrane, tout en autorisant sa compression pour permettre le fonctionnement du dispositif. De manière alternative, les fonctions du capuchon protecteur et du ressort d'écartement peuvent être réunies dans une même pièce.

Le document US-A-5 207 646 divulgue une seringue d'injection comprenant un corps de seringue muni d'une aiguille, des moyens de préhension, une tige d'injection mobile et une cartouche contenant un produit à injecter.

La présente invention a pour but de proposer des seringues d'injection de type à usage unique permettant de pallier les inconvénients des seringues. dispositifs, et modules connus, et permettant au praticien de réaliser l'opération d'aspiration sans exercer de traction sur la tige d'injection, tout en proposant des systèmes de conception simple et pratiques d'emploi.

Un autre but de la présente invention est de proposer des seringues d'injection de type à usage unique permettant un conditionnement stérile minimal et avantageusement sans emballage du corps de seringue.

Un autre but de la présente invention est de fournir des seringues d'injection de type à usage unique permettant grâce à l'aspiration sans traction de diminuer le conditionnement stérile des seringues d'injection proposés.

Un autre but de la présente invention est de proposer des seringues d'injection de type à usage unique selon des coûts peu élevés compatibles notamment avec la destination à usage unique de ces produits.

Un antre but de la présente invention est de proposer des seringues d'injection de type à usage unique procurant à l'utilisateur ainsi qu'aux tiers une ex-cellente sécurité contre les piqûres accidentelles avec l'aiguille d'injection.

La présente invention à cet effet consiste en une seringue d'injection de type à usage unique, notamment pour le domaine dentaire, comprenant :
- un corps de seringue muni d'une aiguille à une première extrémité de celui-ci, ladite aiguille comportant une partie extérieure s'étendant à l'extérieur dudit corps et une partie intérieure s'étendant à l'intérieur dudit corps, ladite aiguille formant une extrémité extérieure au dit corps et une extrémité intérieure au dit corps,
- des moyens de préhension dudit corps de seringue, à une deuxième extrémité de celui-ci opposée à ladite première extrémité,
- une tige d'injection mobile,
- une cartouche contenant un produit à injecter, disposée à l'intérieur dudit corps entre ladite extrémité intérieure de l'aiguille et ladite deuxième extrémité du corps dans une position de repos, ladite cartouche étant apte à se déplacer de la position de repos vers ladite première extrémité du corps sous une pression de la tige d'injection,
- des moyens élastiques permettant un rappel automatique de la cartouche vers ladite position de repos lorsque la pression sur ladite tige d'injection est relâchée, afin de provoquer une dépression dans ladite cartouche, caractérisée en ce que lesdits moyens élastiques sont disposés à l'intérieur dudit corps entre la première extrémité de celui-ci et ladite cartouche, et en ce que lesdits moyens élastiques sont constitués par une enveloppe tubulaire souple entourant ladite partie intérieure de l'aiguille.

Les moyens élastiques tels que définis ci-dessus permettent grâce à un rappel induit par l'élasticité d'un matériau approprié d'éviter à l'utilisateur tout effort de traction pour réaliser l'opération d'aspiration, nécessaire pour vérifier que l'aiguille n'est pas placée dans un vaisseau sanguin. Ainsi, le praticien n'a plus qu'un seul sens d'effort à appliquer lors de la réalisation d'une injection, que se soit pour l'injection elle-même du produit ou bien pour l'opération d'aspiration. En effet, lorsque le praticien désire vérifier si l'extrémité extérieure de l'aiguille a pénétré dans un vaisseau sanguin, il lui suffit de relâcher la pression exercée sur la tige d'injection, les moyens élastiques rappelant alors la cartouche vers la position de repos avant injection provoquant ainsi une dépression dans la cartouche elle-même, aspirant de ce fait le sang par l'extrémité de l'aiguille engagée dans un vaisseau sanguin le cas échéant.

Selon une autre caractéristique avantageuse, lesdits moyens élastiques sont guidés par ladite partie intérieure de l'aiguille.

Selon une autre caractéristique avantageuse, la seringue comprend des moyens amovibles de recouvrement de la partie extérieure de l'aiguille au moins, destinés à être retirés avant l'injection, et ladite enveloppe tubulaire souple recouvre ladite partie intérieure de l'aiguille de manière à définir un espace stérile incluant l'aiguille, à l'intérieur de l'enveloppe tubulaire souple et des moyens de recouvrement.

Cette caractéristique permet d'éviter un conditionnement stérile de la seringue complète, et de confiner l'aiguille seulement dans une enceinte stérile, ce qui diminue d'autant les coûts du conditionnement stérile. On notera la fonction double que joue l'enveloppe tubulaire dans ce cas.

Selon une autre caractéristique avantageuse, la seringue comprend un étui protecteur mobile le long dudit corps de seringue, entre deux positions extrêmes dont l'une permet un recouvrement de ladite partie extérieure de l'aiguille et l'autre un dégagement de la partie extérieure de l'aiguille.

Cette caractéristique procure une excellence sécurité pour le praticien et les tiers contre les piqûres accidentelles avec l'aiguille souillée après injection. En effet, une fois l'injection réalisée, le praticien déplace l'étui en position de recouvrement de la partie extérieure de l'aiguille, évitant ainsi à quiconque la possibilité de se piquer accidentellement en manipulant la seringue.

L'invention sera mieux comprise, et d'autres caractéristiques et avantages apparaîtront à la lecture de la description qui suit d'un exemple de mode de réalisation d'une seringue, d'un dispositif, et d'un module selon l'invention, accompagnée des dessins annexés, exemples donnés à titre d'illustration et sans qu'aucune interprétation restrictive de l'invention ne puisse en être tirée.
La figure 1 représente une vue en coupe longitudinale partielle et en position de repos, avant injection, d'un exemple de mode de réalisation d'une seringue selon l'invention.
La figure 2 représente une vue en coupe longitudinale et en position de travail, en cours d'injection, de l'exemple de la figure 1.
La figure 3 représente une vue en coupe longitudinale après injection de l'exemple de la figure 1.
La figure 4 représente une vue en coupe longitudinale partielle, et en éclaté d'un exemple de mode de réalisation d'une seringue selon l'invention.
La figure 5 représente une vue en coupe longitudinale d'un exemple de mode de réalisation d'un module.

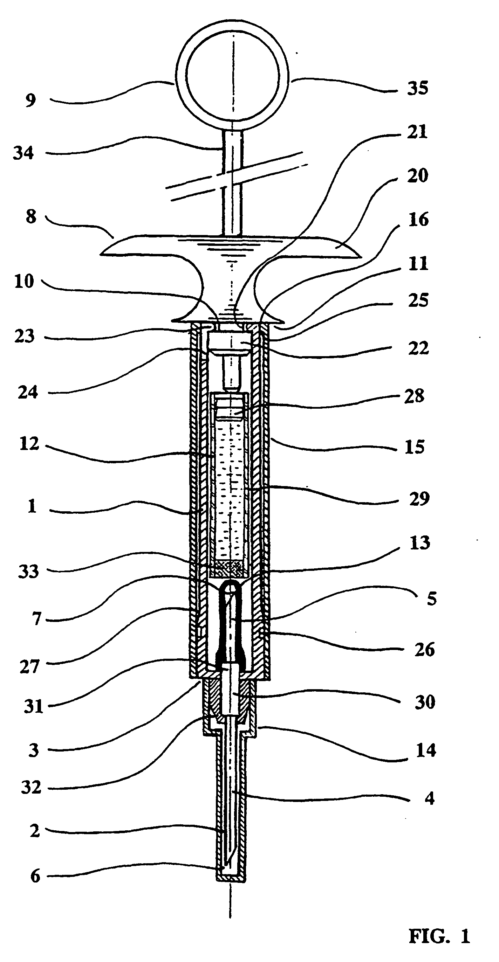

La seringue représentée sur les figures 1, 2, et 3 comporte de manière connue un corps 1 de seringue tubulaire, par exemple de section circulaire, en matière plastique avantageusement transparente, muni d'une aiguille 2 à une première 3 extrémité de celui-ci, l'aiguille 2 comportant une partie extérieure 4 s'étendant à l'extérieur du corps 1 et une partie intérieure 5 s'étendant à l'intérieur du corps 1, et formant une extrémité extérieure 6 au corps 1 et une extrémité intérieure 7 au corps 1, des moyens de préhension 8 du corps 1 de seringue à une deuxième 11 extrémité de celui-ci opposée à la première extrémité 3, une tige d'injection 9 mobile, une cartouche 12 contenant un produit à injecter, disposés à l'intérieur du corps 1 entre l'extrémité intérieure 7 de l'aiguille 2 et la deuxième extrémité 11 du corps 1 dans une position de repos, la cartouche 12 étant apte à se déplacer de la position de repos vers la première 3 extrémité du corps 1 sous une pression de la tige d'injection 9, et comprend selon l'invention des moyens élastiques 13 permettant un rappel automatique de la cartouche 12 vers la position de repos lorsque la pression sur la tige d'injection 9 est relâchée.

Les moyens de préhension sont avantageusement démontables du corps 1 de seringue et sont constitués d'un élément de maintien 8 de toute forme appropriée connue, par exemple comme celle représentée sur les figures 1 à 4, destiné à être assemblé au corps 1 par emboîtement 10 à la deuxième extrémité 11 du corps 1 après introduction de la cartouche 12 dans le corps 1.

L'emboîtement 10 peut être réalisé aux moyens d'une partie cylindrique 22 formée sur l'élément de maintien 8 comme représenté sur la figure 1 par exemple, la partie cylindrique 22 étant d'un diamètre extérieur sensiblement égal au diamètre intérieur du corps 1, et destinée à être introduite dans le corps 1 de seringue afin de réaliser un guidage et un centrage de l'élément de maintien 8 sur le corps 1, de la deuxième extrémité du corps 1 dans laquelle pénètre la partie cylindrique 22, d'une gorge 21 annulaire formée dans la partie cylindrique 22, et d'une nervure annulaire 23 de forme complémentaire à la gorge 21 réalisée à la surface intérieure cylindrique du corps 1 à sa deuxième extrémité 11, la nervure annulaire 23 étant destinée à pénétrer dans la gorge 21 lors de l'introduction de la partie cylindrique 22 de l'élément de maintien 8 dans le corps 1, et ainsi associer ce dernier au corps, comme représenté sur les figures 1, 2, et 3.

Afin de faciliter l'introduction de la partie cylindrique 22 dans le corps 1 de seringue, deux ou trois rainures 24 par exemple sont réalisées dans le corps 1 de manière longitudinale à sa deuxième extrémité 11, les rainures 24 séparant la nervure 23 en deux ou trois secteurs distincts. Les rainures 24 permettent un écartement des lèvres ainsi crées à la deuxième extrémité du corps 1, lors du passage de la partie cylindrique 22, les lèvres venant se resserrer autour de la partie cylindrique 22 sous l'effet de leur élasticité dès que la gorge 21 se trouve dans le plan de la nervure 23 avantageusement lorsque les moyens de préhension sont en butée sur l'extrémité du corps.

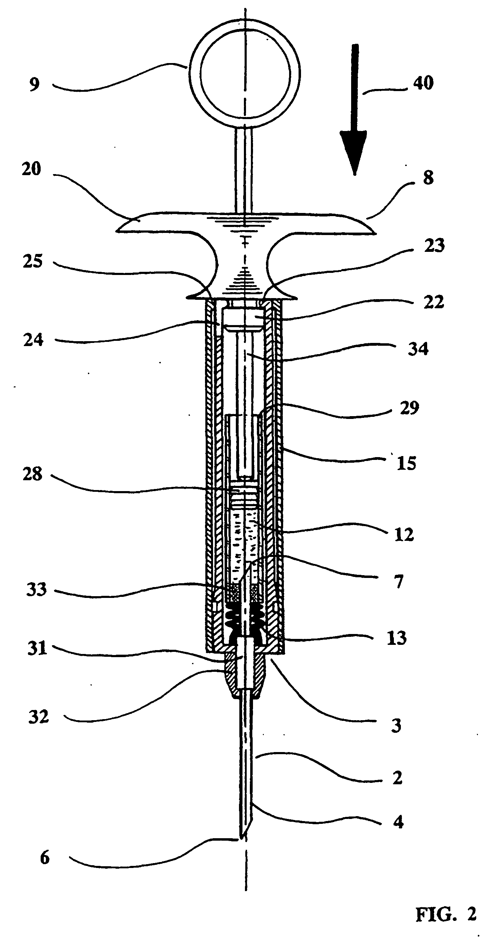

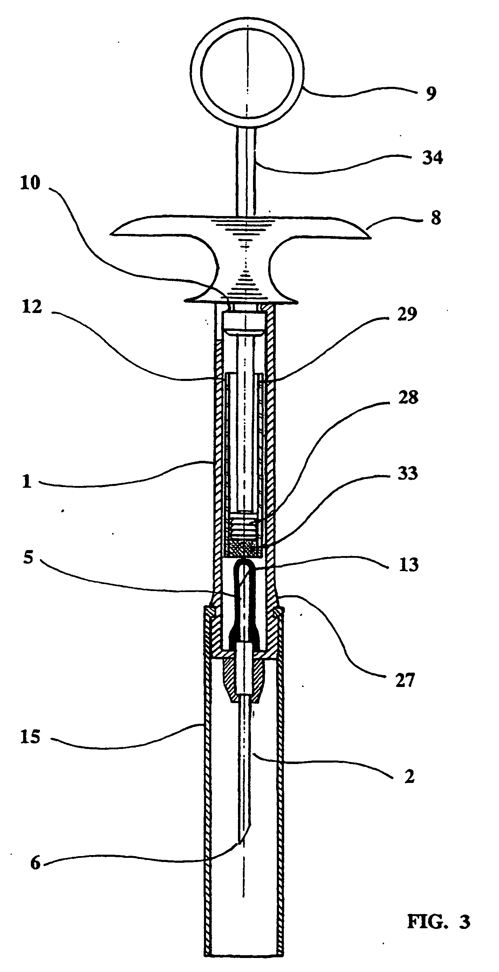

La seringue représentée sur les figures 1, 2, et 3 comprend avantageusement un étui protecteur 15 mobile le long du corps 1 de seringue, entre deux positions extrêmes dont l'une représentée sur la figure 3 permet un recouvrement de la partie extérieure 4 de l'aiguille 2 et l'autre représentée sur les figures 1 et 2, un dégagement de la partie extérieure 4 de l'aiguille 2.

L'étui protecteur 15 dans la position de dégagement de la partie extérieure 4 de l'aiguille 2 sert avantageusement de moyens de verrouillage 16 de l'emboîtement 10. L'étui 15 est muni à une extrémité d'une collerette 25 intérieure dont le diamètre intérieur 16 est légèrement inférieur au diamètre extérieur du corps 1 de manière à maintenir la deuxième extrémité du corps en appui sur la partie cylindrique de l'élément de maintien 8, et empêcher un écartement des lèvres de la deuxième 11 extrémité du corps 1, et la libération de l'emboîtement 10 par exemple sous l'effet des efforts engendrés par le déplacement de la tige 9 lors d'une injection.

La collerette 25 est également destinée à coopérer avec une gorge annulaire 26 réalisée à la surface extérieure du corps 1 et vers sa première extrémité 3, comme représenté sur les figures, afin de permettre une immobilisation de l'étui en position de recouvrement de la partie extérieure 4 de l'aiguille 2 après que l'injection ait été réalisée, comme représenté sur la figure 3, la collerette 25 venant se loger dans la gorge annulaire 26. L'étui 15 dans cette position ne doit plus pouvoir changer de position, tout au moins selon des efforts normaux, et le corps 1 comportera avantageusement, afin de faciliter la pénétration de la collerette 25 dans la gorge 26, une rampe annulaire 27 tronconique destinée à étirer de manière élastique la collerette 25 avant que celle-ci ne se rétracte dans la gorge 26 sous l'effet de l'élasticité du matériau la composant, lequel sera de préférence un matériau plastique transparent. Le corps 1 pourra comporter également en vue de faciliter le placement de l'écui 15 en position de recouvrement de la partie extérieure de l'aiguille 2, des fentes longitudinales (non représentées) réalisées dans la zone de la rampe 27 et/ou de la gorge 26.

L'étui 15 possédera une longueur suffisante pour recouvrir en totalité la partie extérieure 4 de l'aiguille 2 lorsque la collerette 25 et engagée dans la gorge 26.

Les moyens de préhension posséderont une largeur avantageusement constante et sensiblement égale au diamètre extérieur de l'étui protecteur 15.

La tige d'injection 9 est formée d'un axe 34 guidé dans ses déplacements en translation et en rotation par les moyens de préhension 8 par l'intermédiaire d'un alésage réalisé dans ces derniers, alésage dans lequel l'axe 34 est ajusté glissant. Une extrémité de l'axe 34 est muni par exemple d'un anneau de manoeuvre 35 solidaire, et son autre extrémité peut être pourvue de tout élément ou forme approprié en fonction du piston de la cartouche sur lequel elle est destinée à s'appuyer. Une forme en simple surface d'appui, assurant la transmission des efforts nécessaires au déplacement du piston en vue d'évacuer le contenu de la cartouche, est suffisante dans le cas de la seringue, comme représenté sur les figures 1 à 3.

La cartouche 12 se présente sous la forme d'une cartouche connue couramment utilisée dans les seringues à usage dentaire notamment. La cartouche 12 est constituée d'un tube 29 en matériau rigide transparent muni à une extrémité d'un piston 28 apte à se déplacer à l'intérieur du tube 29 pour évacuer le liquide contenu par l'autre extrémité du tube 29 qui est munie d'un bouchon 33 étanche fixe destiné à être percé par la partie intérieure 5 de l'aiguille 2 lors du déplacement de la cartouche dans le corps, de sa deuxième 11 extrémité vers sa première 3 extrémité sous une pression de la tige 9 de seringue en vue d'une injection. La cartouche 12 est introduite dans le corps 1 de seringue par la première extrémité de celui-ci.

Le corps 1 est muni à sa première extrémité d'une aiguille 2 associée à celui-ci selon tout moyen connu, par exemple par collage, soudage, ou analogue, et avantageusement par l'intetmédiaire d'un manchon 30 rigide dont une extrémité 31 émerge à l'intérieur du corps 1, comme représenté sur les figures 1 à 3, extrémité 31 sur laquelle viendront se fixer les moyens élastiques de rappel automatique de la cartouche 12, comme cela va être expliqué ci-dessous.

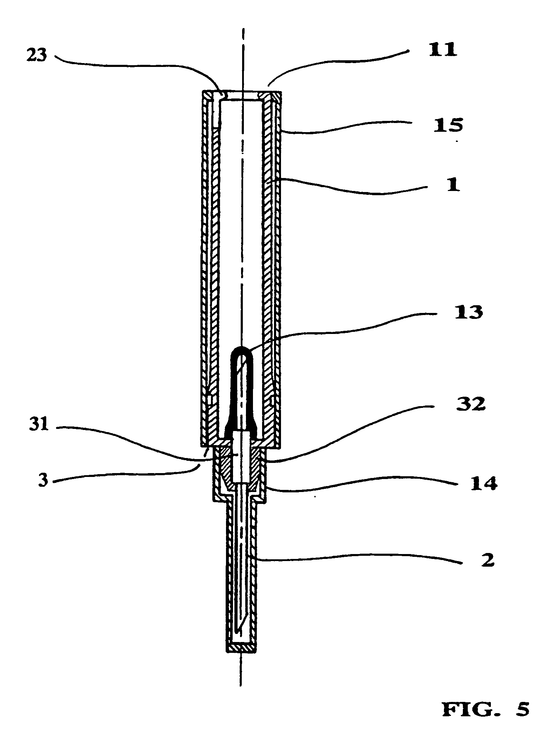

La première extrémité 3 du corps 1 sera munie d'un embout 32 entourant la partie du manchon 31 extérieure au corps 1 de seringue, et sera destinée avec l'embout 32, à coopérer avec des moyens amovibles de recouvrement 14 au moins de la partie extérieure 4 de l'aiguille 2, en vue de confiner celle-ci dans un espace stérile jusqu'au retrait des moyens amovibles de recouvrement 14 pour réaliser l'injection. Les moyens amovibles de recouvrement adopteront la forme d'un capuchon 14 étanche, par exemple en matière plastique, dont une extrémité ouverte adopte une forme intérieure complémentaire de la forme extérieure de l'embout 32 du corps 1, sur lequel le capuchon 14 est destiné à être emmancher en force, comme représenté sur les figures 1 et 5.

Les moyens élastiques sont avantageusement disposés à l'intérieur du corps 1 entre la première 3 extrémité de celui-ci et la cartouche 12, et sont constitués par une enveloppe tubulaire 13 souple entourant la partie intérieure 5 de l'aiguille 2, comme représenté sur les figures 1 et 3. L'enveloppe tubulaire 13 sera de préférence réalisée dans un matériau élastique de type caoutchouc, naturel ou synthétique, possédant de bonne qualité d'élasticité afin de permettre un rappel automatique de la cartouche vers la deuxième extrémité du corps 1 de seringue dès que la pression sur la tige 9 de seringue est relâchée.

L'enveloppe tubulaire 13 sera de préférence étanche, et recouvrira la partie intérieure 5 de l'aiguille 2, de manière à définir un espace stérile incluant l'aiguille 2, à l'intérieur de l'enveloppe tubulaire souple et des moyens de recouvrement 14. L'enveloppe 13 est avantageusement fixée sur la partie du manchon 30 émergeant à l'intérieur du corps 1, comme représenté sur les figures 1 à 3, par l'intermédiaire d'un emmanchement forcé utilisant l'élasticité de l'enveloppe 13 pour enserrer fermement la partie émergeant du manchon 30. La mise en place de l'enveloppe 13 se fait par pression sur celle-ci tout en veillant à ne pas la perforer sur l'extrémité 7 intérieure de l'aiguille 2.

Les figures 1, 2, et 3 montrent la position de la cartouche 12 et du piston 28 de celle-ci, respectivement avant utilisation de la seringue prête pour une injection, en cours d'injection, et après l'injection l'opérateur ayant manuellement placer l'étui 15 dans la position de recouvrement de la partie extérieure de l'aiguille 2.

Le fonctionnement des moyens de rappel automatique de la cartouche est le suivant :
l'enveloppe 13 avant utilisation de la seringue, telle que représenté sur la figure 1, permet avantageusement et outre le confinement stérile de l'aiguille, de former entre la cartouche et l'extrémité intérieure 7 de l'aiguille un film amortisseur évitant toute perforation accidentelle du bouchon étanche 33 de la cartouche 12, lors des manipulations ou transports de la seringue ;
sous une pression de la tige 9 de seringue, représentée par la flèche 40 sur la figure 2, la cartouche 12 se déplace vers la première extrémité 3 du corps 1 de seringue, et l'extrémité 7 intérieure de l'aiguille 2 vient inciser l'enveloppe 13 et le bouchon étanche 33 de la cartouche 12. L'enveloppe 13 est alors en butée entre la cartouche 12 et l'extrémité 3 du corps 1 ou le manchon 30, et toute nouvelle pression, ou pression continue, sur la tige 9 comprimera l'enveloppe 13, guidée dans son déplacement en compression par la partie intérieure 5 de l'aiguille 2, comme représenté sur la figure 2 ;
dès que la pression sur la tige 9 est relâchée, le potentiel d'énergie emmagasiné par l'enveloppe 13 comprimée se libère et permet de déplacer la cartouche 12 ainsi que la tige 9 vers sa position initiale, créant une dépression à l'intérieur de la cartouche 12 provoquant ainsi une aspiration par l'extrémité extérieure 6 de l'aiguille 2. On veillera que la partie intérieure 5 de l'aiguille 2 soit suffisamment longue pour permettre un déplacement de la cartouche 12, nécessaire à une aspiration suffisante pour l'introduction d'une faible quantité de sang dans la cartouche 12 le cas échéant. L'opérateur contrôle avantageusement, par une immobilisation appropriée de la tige 9, la valeur du déplacement de la cartouche 12 vers sa position initiale ;
lorsque l'injection est terminée, l'enveloppe 13 rappelle automatiquement et avantageusement la cartouche dans sa position initiale, comme représenté sur la figure 3, sous l'effet de l'énergie emmagasinée au cours de l'injection. L'enveloppe 13 reprend sensiblement, sous l'effet de son élasticité, sa forme initiale avant injection, l'incision créée pour l'injection se refermant d'elle-même par jonction des lèvres de l'enveloppe. La partie intérieure de l'aiguille est ainsi totalement recouverte après injection, conférant à la seringue selon l'invention une sécurité accrue ; de même, si lors de l'injection la cartouche venait à se dégager de la partie intérieure 5 de l'aiguille 2, par exemple par relâchement complet de la pression sur la tige 9, l'enveloppe 13 recouvrirait aussitôt cette dernière sous l'effet de son élasticité comme expliqué ci-dessus, empêchant toute contamination de l'aiguille par le milieu extérieur.

Il est à noter que les moyens de préhension 8, qui sont avantageusement démontables dans l'exemple de mode de réalisation de type à usage unique de la seringue selon l'invention, peuvent également être fixés de manière non démontable au corps de seringue, comme cela est le cas dans des seringues connues dans lesquelles la cartouche est introduite dans le corps de seringue par une fenêtre latérale au corps.

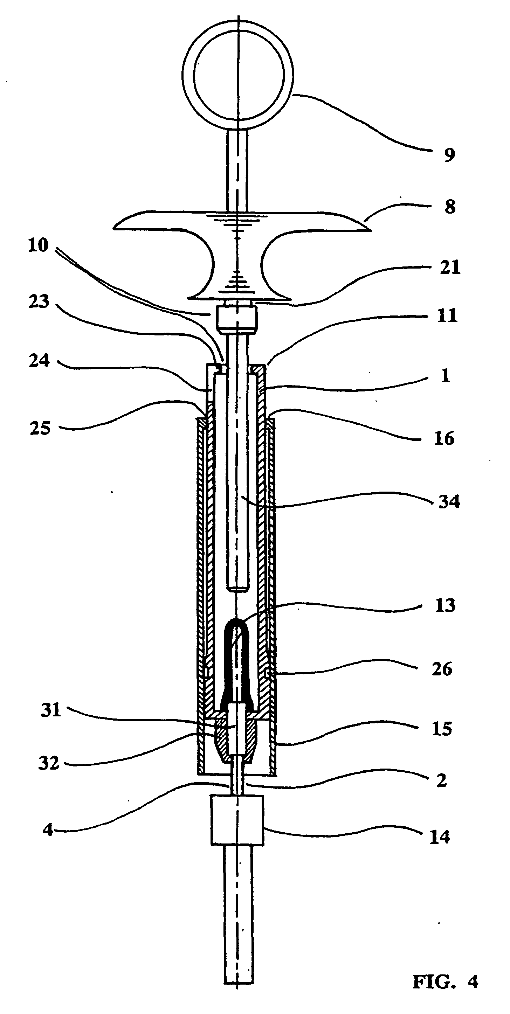

La figure 4 représente un exemple de mode de réalisation d'un dispositif d'injection à usage unique, en tout point identique à la seringue selon l'invention décrite à l'aide des figures 1 à 3, mais dans laquelle la cartouche 12 a été retirée. Les références utilisées pour les éléments du dispositif sont identiques aux références utilisées pour les mêmes éléments de la seringue. Un tel dispositif est conditionné sans cartouche, l'utilisateur ou le distributeur introduisant la cartouche de son choix.

Sur la figure 4, les moyens de préhension 8 avec la tige 9 les traversant libre en translation et en rotation, ont été représentés non assemblés au corps 1 de seringue. Le capuchon 14 a également été représenté sur la figure 4 en étant non associé au corps 1, et l'étui 15 a été représenté dans une position permettant l'emboîtement des moyens de préhension 8 sur le corps 1 de seringue comme expliqué plus haut à l'aide des figures 1 à 3.

On remarquera que lorsque le capuchon 14 est en place sur l'embout 32, sur la seringue comme sur le dispositif selon l'invention, l'aiguille 2 se trouve confinée dans un espace fermé, avantageusement stérile, permettant d'éviter un conditionnement de tout le dispositif ou de toute la seringue, coûteux et inutile, par exemple sous emballage étanche stérile.

Pour se trouver en condition d'utilisation avant injection, le dispositif représenté sur la figure 4 doit subir les opérations suivantes : introduction d'une cartouche (non représentée) dans le corps 1 par sa deuxième extrémité 11, mise en place des moyens de préhension 8 et de la tige 9 sur la deuxième extrémité 11 du corps 1, mise en place de l'étui 15 dans sa position de dégagement de la partie extérieure 4 de l'aiguille 2 afin notamment de verrouiller les moyens de préhension sur le corps 1, et retrait du capuchon 14.

La figure 5 représente un exemple de mode de réalisation d'un module d'injection à usage unique, en tout point identique au dispositif décrit à l'aide de la figure 4, mais dans lequel les moyens de préhension et la tige d'injection ont été retirés. Les références utilisées pour les éléments du module sont identiques aux références utilisées pour les mêmes éléments du dispositif. Un tel module est conditionné sans moyens de préhension et sans tige d'injection, l'utilisateur ou le distributeur pouvant utiliser les moyens de préhension et la tige d'injection réutilisables, de son choix.

Sur la figure 5, le module a été représenté tel que conditionné pour l'utilisation ou la distribution. On remarquera que, comme pour le dispositif, un conditionnement complet du module est évité par un confinement de l'aiguille 2 dans un espace stérile à l'intérieur de l'enveloppe tubulaire 13 souple et des moyens de recouvrement 14.

L'intérêt du module est de permettre de réduire éventuellement les coûts de revient d'une seringue selon l'invention, en évitant de fabriquer des moyens de préhension et une tige d'injection pour chaque module produit, l'utilisateur disposant dans ce cas de moyens de préhension et d'une tige appropriés, par exemple en acier inoxydable, et donc réutilisables.

Dans le cas du dispositif, les moyens de préhension et la tige d'injection sont destinés à un usage unique, et sont fabriqués dans un matériau peu coûteux, en matière plastique par exemple.

## Revendications

1. Seringue d'injection de type à usage unique, notamment pour le domaine dentaire, comprenant :
- un corps (1) de seringue muni d'une aiguille (2) à une première (3) extrémité de celui-ci, ladite aiguille comportant une partie extérieure (4) s'étendant à l'extérieur dudit corps et une partie intérieure (5) s'étendant à l'intérieur dudit corps, ladite aiguille formant une extrémité extérieure (6) au dit corps et une extrémité intérieure (7) au dit corps,
- des moyens de préhension (8) dudit corps de seringue, à une deuxième (11) extrémité de celui-ci opposée à ladite première extrémité,
- une tige d'injection (9) mobile,
- une cartouche (12) contenant un produit à injecter, disposée à l'intérieur dudit corps entre ladite extrémité intérieure de l'aiguille et ladite deuxième extrémité du corps dans une position de repos, ladite cartouche étant apte à se déplacer de la position de repos vers ladite première (3) extrémité du corps sous une pression de la tige d'injection,
- des moyens élastiques permettant un rappel automatique de la cartouche vers ladite position de repos lorsque la pression sur ladite tige d'injection est relâchée, afin de provoquer une dépression dans ladite cartouche,
***caractérisée en ce que*** lesdits moyens élastiques (13) sont disposés à l'intérieur dudit corps (1) entre la première (3) extrémité de celui-ci et ladite cartouche (12), et **en ce que** lesdits moyens élastiques sont constitués par une enveloppe tubulaire souple entourant ladite partie intérieure (5) de l'aiguille.

2. Seringue selon la revendication 1, ***caractérisée en ce que*** lesdits moyens élastiques (13) sont guidés par ladite partie intérieure (5) de l'aiguille.

3. Seringue selon la revendication 1 ou 2, ***caractérisée en ce qu'***elle comprend des moyens amovibles de recouvrement (14) de la partie extérieure (4) de l'aiguille (2) au moins, destinés à être retirés avant l'injection, et **en ce que** ladite enveloppe tubulaire (13) souple recouvre ladite partie intérieure de l'aiguille, de manière à définir un espace stérile incluant l'aiguille (2), à l'intérieur de l'enveloppe tubulaire souple et des moyens de recouvrement.

4. Seringue selon l'une quelconque des revendications 1 à *3, **caractérisée en ce qu*****'**elle comprend un étui protecteur (15) mobile le long dudit corps (1) de seringue, entre deux positions extrêmes dont l'une permet un recouvrement de ladite partie extérieure (4) de l'aiguille et l'autre un dégagement de la partie extérieure de l'aiguille.

5. Seringue selon la revendication 4, ***caractérisée en ce qu'***elle comprend des moyens d'emboîtement (10) desdits moyens de préhension (8) dans ledit corps (2) de seringue à la deuxième extrémité (11) de celui-ci, des moyens de verrouillage (16) desdits moyens d'emboîtement via l'étui protecteur (15) dans la position de dégagement de ladite partie extérieure de l'aiguille.

## Patentansprüche

1. Injektionsspritze für eine einmalige Verwendung, insbesondere für den zahnärztlichen Bereich, mit
- einem Spritzenkörper (1), der an einem ersten Ende (3) mit einer Nadel (2) versehen ist, und die Nadel ein Außenteil (4), das sich in den Außenbereich des Körpers erstreckt, und ein Innenteil (5), das sich in den Innenbereich des Körpers erstreckt, aufweist, wobei die Nadel ein äußeres Ende (6) und ein inneres Ende (7) an dem Körper bildet,
- Greifmitteln (8) des Spritzenkörpers an dessen gegenüber dem ersten Ende liegendem zweiten Ende (11),
- einem beweglichen Injektionsstift (9),
- einer Patrone (12), die ein zu injizierendes Mittel enthält, die im Inneren des Körpers zwischen dem innen liegenden Ende der Nadel und dem zweiten Ende des Körpers in einer Ruhestellung angeordnet ist, wobei die Patrone unter Druck des Injektionsstiftes aus der Ruhestellung in das erste Ende (3) des Körpers verschiebbar ist,
- elastischen Mitteln, die eine automatische Rückstellung der Patrone in die Ruhestellung erlauben, wenn der Druck auf den Injektionsstift entspannt wird, um einen Unterdruck in der Patrone zu erzeugen,
**dadurch gekennzeichnet,**
**dass** die elastischen Mittel (13) im Innenbereich des Körpers (1) zwischen dessen erstem Ende (3) und der Patrone (12) angeordnet sind, und
**dass** die elastischen Mittel durch eine biegsame röhrenförmige Hülle gebildet werden, die das Innenteil (5) der Nadel umgibt.

2. Spritze nach Anspruch 1,
**dadurch gekennzeichnet,**
**dass** die elastischen Mittel (13) durch das Innenteil (5) der Nadel geführt werden.

3. Spritze nach Anspruch 1 oder 2,
**dadurch gekennzeichnet,**
**dass** sie zumindest abnehmbare Abdeckmittel (14) des Außenteils (4) der Nadel (2) aufweist, die dazu vorgesehen sind, vor der Injektion abgezogen zu werden, und
**dass** die biegsame röhrenförmige Hülle (13) das Innenteil der Nadel derart abdeckt, um einen die Nadel (2) einschließenden sterilen Raum im Innenbereich der biegsamen röhrenförmigen Hülle und der Bedeckungsmittel abzugrenzen.

4. Spritze nach einem der Ansprüche 1 bis 3,
**dadurch gekennzeichnet,**
**dass** sie eine entlang des Spritzenkörpers (1) zwischen zwei Extremstellungen bewegliche Schutzhülle 1(15) aufweist, wovon die eine Extremstellung eine Bedeckung des Außenteils (4) der Nadel und die andere Extremstellung einen Austritt des Außenteils der Nadel erlaubt.

5. Spritze nach Anspruch 4,
**dadurch gekennzeichnet,**
**dass** sie in dem Spritzenkörper (2) an dessen zweitem Ende (11) Einfügungsmittel (10) der Greifmittel (8) und in der Austrittsstelle des Außenteils der Nadel durch die Schutzhülle (15) Verriegelungsmittel (16) der Einfügungsmittel aufweist.

## Claims

1. Injection syringe of the type for single use, especially for the field of dentistry, comprising:
- a syringe body (1) fitted with a needle (2) at a first end (3) of the former, said needle comprising an external portion (4) extending outside said body and an internal portion (5) extending inside said body, said needle forming an end external (6) to said body and an end (7) internal to said body,
- means for grasping (8) said syringe body, at a second end (11) of the former, opposite said first end,
- a mobile injection rod (9),
- a cartridge (12) containing a project to be injected, disposed inside said body between said internal end of the needle and said second end of the body in an inoperative position, said cartridge being capable of moving from the inoperative position towards said first end (3) of the body under the pressure of the injection rod,
- elastic means (13) for automatically returning the cartridge towards said inoperative position when the pressure on said injection rod is released, in order to create a depression in said cartridge,
**characterised in that** said elastic means (13) are disposed inside said body (1) between the first end (3) of the latter and said cartridge (12), and **in that** said elastic means are formed by a flexible tubular envelope surrounding said internal portion (5) of the needle.

2. Syringe according to claim 1, **characterised in that** said elastic means (13) are guided by said internal portion (5) of the needle.

3. Syringe according to claim 1 or 2, **characterised in that** it comprises detachable means for covering (14) at least the external portion (4) of the needle (2), these means being intended to be removed before the injection, and **in that** said flexible tubular envelope (13) covers said internal portion of the needle, in such a way as to define a sterile space enclosing the needle (2), inside the flexible tubular envelope and the covering means.

4. Syringe according to any one of claims 1 to 3, **characterised in that** it comprises a protective sheath (15) moveable along said syringe body (1) between two extreme positions, one of which permits covering said external portion (4) of the needle, and the other the disengagement of the external portion of the needle.

5. Syringe according to claim 4, **characterised in that** it comprises means of housing (10) said grasping means (8) in said syringe body (2) at the second end (11) of the latter, means of locking (16) said housing means via the protective sheath (15) in the disengaged position of said external portion of the needle.
